# EUROPEAN PATENT APPLICATION

(11) **EP 1 734 110 A1**
(43) Date of publication of application: **20.12.2006**
(21) Application number: 05720315.0
(22) Date of filing: 09.03.2005
(51) Int. Cl.: C12M 3/00

(54) **CULTURE DEVICE**

(30) Priority: 11.03.2004 JP 2004068782
(71) Applicant: Nagoya Industrial Science Research Institute, Nagoya-shi, Aichi 460-0008 (JP)
(72) Inventor: NARUSE, Keiji, Chikusa-ku, Nagoa-shi, Aichi 4640033 (JP); ISHIDA, Norio, Osaka-shi, Osaka 5460002 (JP)
(74) Representative: Müller Fottner Steinecke
(86) International application number: PCT/JP2005/004043
(87) International publication number: WO 2005/087913

(57) **Abstract**

A culture device to apply uniform stress to cells is proposed. A culture device is formed in a rectangular box shape from a deformable material, comprising a bottom membrane and side walls upstanding from the entire periphery of said bottom membrane, wherein an engaging member is formed in opposing side walls on a line extended from a peripheral edge of said bottom membrane. By providing the latching member in the culture membrane so as to latch the cells to the latching member, the slippage between the bottom membrane and sample cells is forestalled upon the extension of the culture membrane.

## Description

### TECHNICAL FIELD

The present invention relates to a culture device for culturing sample cells while applying stress to the sample cells.

### BACKGROUND ART

It is known that when sample cells are cultured while applying stress to the sample cells, the stress serves as an impetus toward specific changes in the cultured cells.
Conventional methods for applying stress to cultured sample cells have been proposed.
For example, a technique of applying stress to sample cells by suctioning the bottom membrane of a dish on which the sample cells are placed so that the bottom membrane deforms is disclosed in Patent Documents 1 through 3.

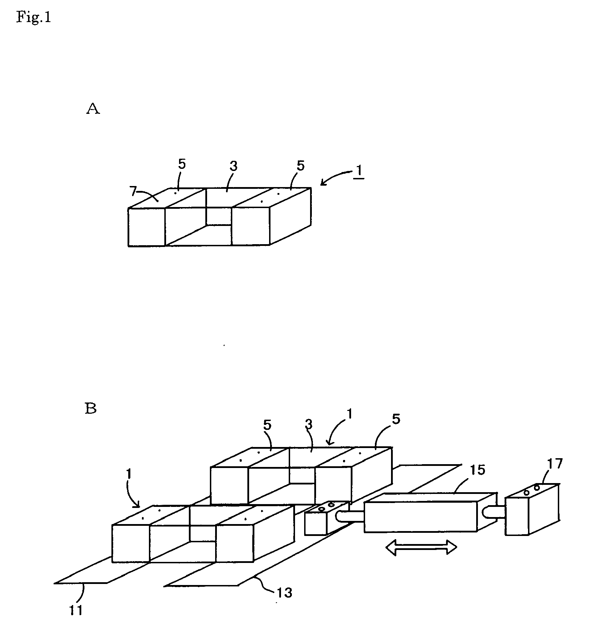

The present inventors, considering it difficult to apply stress uniformly to the sample cells when the bottom membrane of the culture device is deformed using a suction device, have investigated methods of applying tensile stress to a culture device in a parallel direction to the bottom membrane of the culture device (see Non-patent Document 1).
As shown in Fig. 1, in a culture device 1 sample cells are placed on a translucent, deformable culture membrane 3 having a thickness of 200 µm and coated with fibronectin, for example, and side walls 5, 5 (thickness: 400 µm) are provided on the two opposing sides of the culture membrane 3 (see Non-patent Document 1). The thick side walls 5 are employed to ensure that the culture membrane 3 carrying the sample cells is stretched evenly.

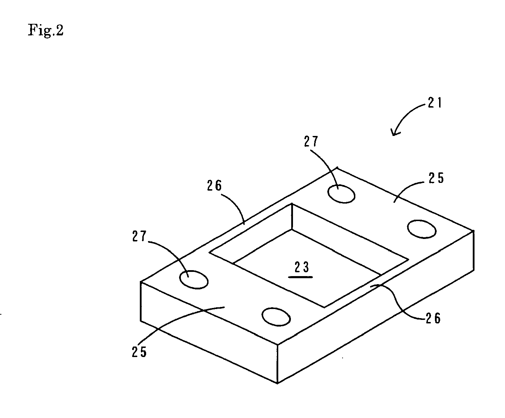

In Fig. 1, engagement holes 7 are formed in the side walls 5.
Pins (not shown) of a stretching device 10 are inserted into the engagement holes 7.
As shown in Fig. 2, the stretching device 10 includes a fixed plate 11, a movable plate 13, a step motor 15, and a control device 17.
Pins are formed in predetermined positions on the fixed plate 11 and movable plate 13, and the pins are inserted into the engagement holes 7 formed in the culture device 1. The movable plate 13 is moved in accordance with the rotation of the step motor 15 such that the distance between the movable plate 13 and fixed plate 11 varies. As a result, the culture membrane 3 of the culture device 1 is stretched, and hence stress is applied to the sample cells. Rotation of the step motor 15 is controlled by the control device 17.

Patent Documents 1 through 6 may also be cited in relation to the present invention.

Patent Document 1: U.S. Patent Publication No. 4,789,601
Patent Document 2: U.S. Patent Publication No. 4,822,741
Patent Document 3: U.S. Patent Publication No. 4,839,280
Patent Document 4: Japanese Patent Application Publication No. 2003-61642
Patent Document 5: U.S. Patent Publication No. 6,107,081
Patent Document 6: International Patent Application Publication WO02/46365
Non-patent Document 1: Involvement of SA channels in orienting response of cultured endothelial cells to cyclic stretch, the American Physiological Society, 1998, H1532-1538, KEIJI NARUSE et al.

### DISCLOSURE OF THE INVENTION

### Problem to be Solved by the Invention

It was found to be possible to apply the desired stress to the cells using the culture device shown in Fig. 1.
However, research and development currently need to be performed under more finely-controlled conditions in the field of cell cultivation. Accordingly, the present inventors have investigated culture devices with which stress can be applied more uniformly to cells.
It is therefore a first object of the present invention to propose a culture device in which extremely uniform stress can be applied to cells.
Another object of the present invention is to propose a culture device in which stress can be applied to cells uniformly in a culture device having a large surface area, which is suitable for cell regeneration and the like.

### Means for Solving the Problem

The present invention has been designed to solve at least one of the problems described above. More specifically, the present invention is a culture device formed in a rectangular box shape from a deformable material, including a bottom membrane and side walls upstanding from the entire peripheral edge of the bottom membrane, wherein an engaging member is formed in a pair of opposing said side walls on a line extended from the peripheral edge of the bottom membrane.

### Effects of the Invention

According to the culture device structured in this manner, the side walls stand upright from the entire periphery of the bottom membrane on which the sample cells are placed, and hence it is possible to prevent the bottom membrane from deforming irregularly and unforeseen stress from being applied to the sample cells. Further, the engaging member to which the force of a stretching device is applied exists in the highly rigid side walls on a line extended from the peripheral edge of the bottom membrane in the stretching direction, and therefore the bottom membrane can be stretched uniformly. In other words, when the bottom membrane is stretched, the peripheral edge thereof in the stretching direction tends to sag at the center, but since force is applied directly to the peripheral edge via the engaging member, this sagging can be prevented. As a result, stress can be applied uniformly to the sample cells on the bottom membrane.

According to another aspect of the present invention, a latching member is provided on the bottom membrane and the sample cells are latched to the latching member. When slippage occurs between the bottom membrane and the sample cells, it may be impossible to apply stress uniformly to the sample cells even when the bottom membrane is stretched uniformly.
Here, by providing the bottom membrane with the latching member such that the sample cells are latched thereto, slippage between the bottom membrane and sample cells can be forestalled. Hence, by stretching the bottom membrane uniformly, stress can be applied uniformly to the sample cells. By providing the latching members in a plurality, slippage between the bottom membrane and sample cells can be prevented over the entire range of the bottom membrane even when the bottom membrane (culture membrane) has a large surface area. A culture membrane having a large surface area is suitable for use in cell regeneration.

### BRIEF DESCRIPTION OF THE DRAWINGS

[Fig. 1] Fig. 1 shows a conventional example of a culture device and a stretching device thereof.
[Fig. 2] Fig. 2 is a perspective view of a culture device according to an embodiment of the present invention.
[Fig. 3] Fig. 3 shows the culture device according to the same embodiment, (A) being a plan view thereof, (B) being a front view thereof, (C) being a bottom view thereof, and (D) being a right side view thereof.
[Fig. 4] Fig. 4 is a sectional view along a line A-A in Fig. 3.
[Fig. 5] Fig. 5 is a sectional view showing a manner of use of the culture device according to the embodiment.
[Fig. 6] Fig. 6 is a perspective view showing a culture device according to another embodiment of the present invention.
[Fig. 7] Fig. 7 shows the culture device according to the same embodiment, (A) being a plan view thereof, (B) being a front view thereof, (C) being a bottom view thereof, and (D) being a right side view thereof.
[Fig. 8] Fig. 8 is a sectional view along a line A-A in Fig. 7.
[Fig. 9] Fig. 9 shows the manner in which a latching member formed on a bottom membrane deforms.

### BEST MODES FOR CARRYING OUT THE INVENTION

Each element of the present invention will be described below in detail.
In one aspect of the present invention, a rectangular box-shaped object is used as a culture device. By forming the culture device in the shape of a rectangular box, transportation and storage of the expendable culture device is facilitated.
The culture device is formed from a deformable material so that stress can be applied to sample cells indirectly by stretching the culture device.
A material which does not interfere chemically with the sample cells, such as silicone elastomer, is used as the material for forming the culture device.

A bottom membrane takes a planar rectangular form, and preferably has a uniform membrane thickness so that it stretches evenly. The bottom membrane is preferably formed using a optical transparent material so that the sample cells can be observed under an optical microscope.
Side walls are preferably provided upstanding from the entire periphery of the bottom membrane. The side walls are formed from thick film to ensure mechanical rigidity. In so doing, the bottom membrane can be prevented from deforming in an unregulated fashion.
The bottom membrane and side walls are preferably formed integrally to reduce the number of components and thereby reduce the manufacturing cost, but the bottom membrane and side walls may be formed separately.

Engaging members are provided in the pair of side walls formed on opposing edges of the rectangular bottom membrane. The engaging members should engage with a fixed plate and a moving plate of a stretching device such that positional variation between these two members, the relative positions of which are controllable, causes the culture device to deform.
Accordingly, protrusions may be provided on the side walls and these protrusions may be engaged with the plates. Furthermore, the two plates may be moved together.

When the bottom membrane is stretched, the peripheral edge thereof in the stretching direction deforms such that a central portion thereof sags. As a result of this deformation, the direction of the stress applied to the sample cells on the deformed part differs from that of the other parts.
Therefore, the engaging members of the present invention are formed on a line extended from the peripheral edge of the rectangular bottom membrane in the stretching direction. In so doing, the force applied to the engaging member is applied to the peripheral edge of the bottom membrane in the stretching direction, and hence this part of the bottom membrane is pulled reliably. As a result, the peripheral edge is prevented from deforming, and uniform stress is applied to all of the sample cells on the bottom membrane.
There are no particular limitations on the type and procurement method of the sample cells used in the culture device of the present invention. For example, vascular endothelial cells (from a human, monkey, pig, cow, rat, mouse, rabbit, and so on), smooth muscle cells, myocardial cells, skeletal muscle cells, fibroblasts, osteoblasts, cartilage cells, osteoclasts, nerve cells, and so on may be used.

### First Embodiment

The present invention will now be described in further detail on the basis of an embodiment.

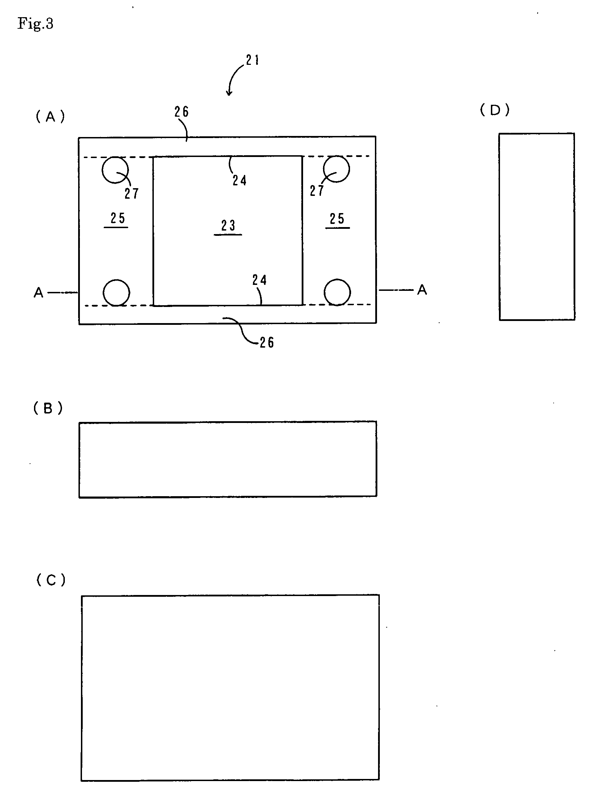

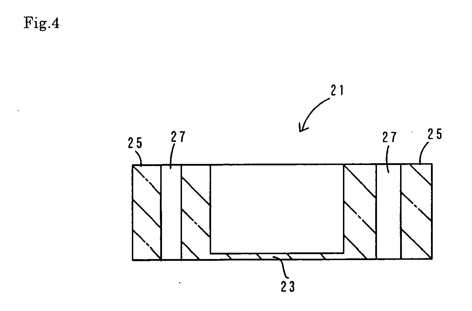

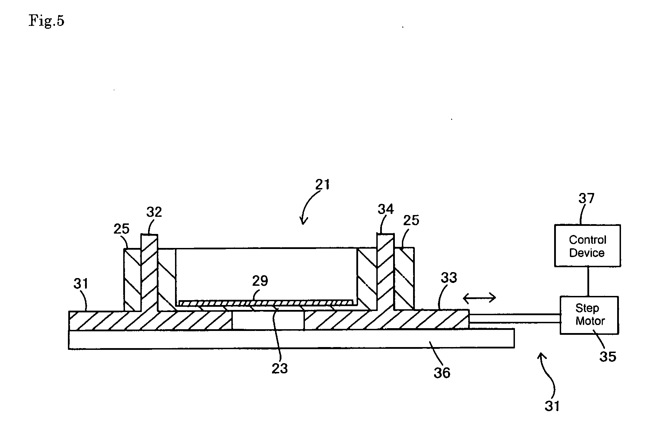

Fig. 2 shows a culture device 21 of this embodiment. Fig. 2 is a perspective view thereof, while Fig. 3(A) is a plan view, Fig. 3(B) is a front view, Fig. 3(C) is a bottom view, and Fig. 3(D) is a right side view. Note that the left side view is identical to Fig. 3(D) and has therefore been omitted. Fig. 4 is a sectional view along a line A-A in Fig. 3(A). Fig. 5 shows a manner of use.
The culture device 21 of this embodiment is a die-molded box made of a transparent silicone elastomer, and includes a thin bottom membrane 23, and side walls 25, 26 formed upstanding integrally from the peripheral edge of the bottom membrane 23. The bottom membrane 23 has a film thickness of approximately 100 µm or approximately 200 µm. The side wall 25 is approximately 1cm thick, and the side wall 26 is approximately 2mm thick. Engagement holes 27 are formed in the side wall 25.
The surface of the bottom membrane 23 is coated with fibronectin, collagen, or the like for implanting cells.

Fig. 5 shows a stretching device 30 of this embodiment. In the stretching device 30, a fixed plate 31 is fixed to a rail plate 36, and a movable plate 33 is disposed slidably on the rail plate 36. Pins 32, 34 protrude from the fixed plate 31 and movable plate 33, respectively, and these pins 32, 34 are inserted into the engagement holes 27 in the culture device 21.
The movable plate 33 moves in the direction shown by an arrow in the drawing via a rod in conjunction with the rotation of a step motor 35. The reference numeral 37 denotes a control device for controlling the rotation of the step motor 35, and in this embodiment a computer is employed as the control device 37.
When the step motor 35 is rotated to move the movable plate 33 in a direction away from the fixed plate 31, the force thereof is transmitted to the side wall 25 of the culture device 21 via the pin 34. As a result, the bottom membrane 23 is stretched in the direction of the line extended from the peripheral edge 24.

At this time, the engagement holes 27 are positioned on the line extended from the peripheral edges 24, 24 of the bottom membrane 23, as shown in Fig. 3(A). More preferably, as shown in the drawing, the line extended matches the outer edge portion of the engagement holes 27. Thus the force generated from the pin 32 to the pin 34 is applied more directly to the peripheral edge 24 of the bottom membrane 23. Hence, sagging of the peripheral edge 24 is prevented and the bottom membrane 23 is stretched evenly, and as a result, the stress applied to sample cells 29 on the bottom membrane 23 is uniform.

### Second Embodiment

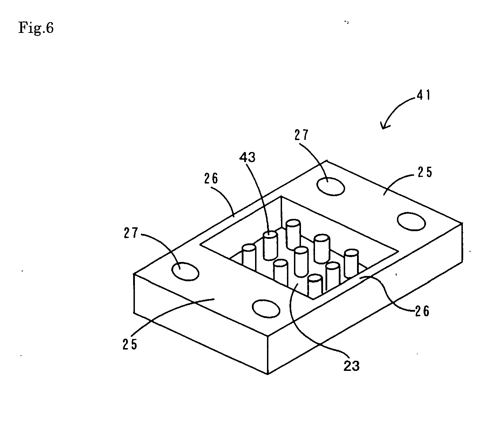

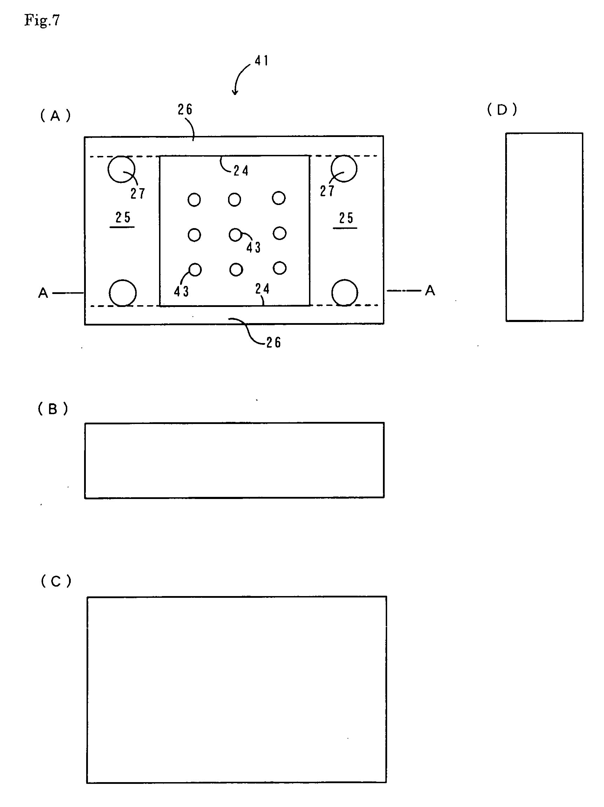

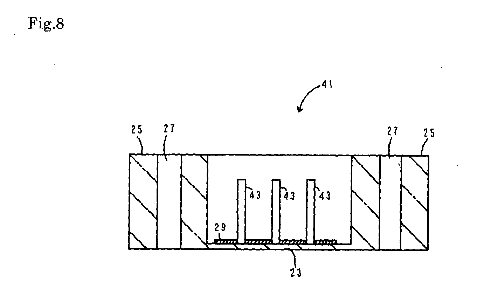

Figs. 6 to 8 show a culture device 41 of another embodiment. Identical elements to those of the previous embodiment have been allocated identical reference numerals, and description thereof has been partially omitted.
In the culture device 41 of this embodiment, protrusions 43 are formed on the bottom membrane 23. The protrusions 43 interfere with the sample cells 29 and thereby prevent slippage between the sample cells 29 and the bottom membrane 23. As a result, uniform stress can be applied to the sample cells 29.
By making the protrusions 43 porous, the latching force between the protrusions and sample cells is improved.

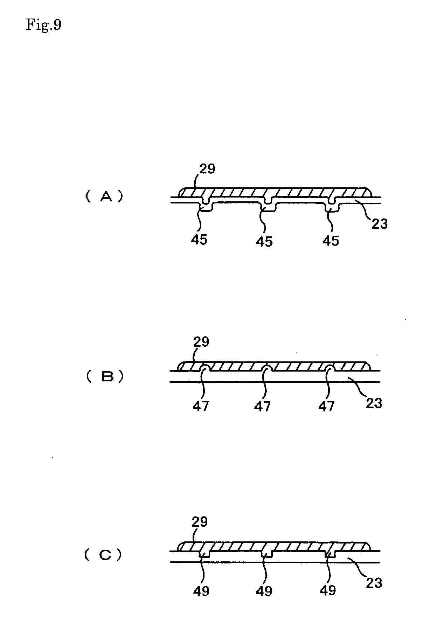

The protrusions 43 latch the sample cells 29 and prevent slippage between the sample cells 29 and the bottom membrane 23.
This action can also be achieved using recessed portions 45 such as those shown in Fig. 9(A), small protrusions 47 such as those shown in Fig. 9(B), and grooves 49 such as those shown in Fig. 9(C). The recessed portions 45, protrusions 47, and grooves 49 may be either continuous or non-continuous in a direction which intersects (preferably a direction orthogonal to) the peripheral edge 24 of the bottom membrane 23.

In this embodiment, a latching member is provided on the bottom membrane, which serves as a culture membrane of the rectangular box-shaped culture device, but there are no particular limitations on the shape and structure of the culture device. For example, the latching member may be provided in a culture device which is deformed by suction.

This invention is not limited to or by the above description of the embodiments of the invention, and includes various modifications that could be arrived at easily by a person skilled in the art and do not depart from the scope of the claims.

## Claims

1. A culture device formed in a rectangular box shape from a deformable material, comprising a bottom membrane and side walls upstanding from the entire periphery of said bottom membrane, wherein an engaging member is formed in opposing side walls on a line extended from a peripheral edge of said bottom membrane.

2. A culture device according to claim 1, **characterized in that** an external edge of said engaging member and said peripheral edge of said bottom membrane are positioned substantially collinearly.

3. A culture device according to claim 1 or claim 2, **characterized in that** a latching member which latches sample cells is formed on said bottom membrane.

4. A culture device according to any of claims 1 through 3, **characterized in that** said bottom membrane and said side walls are integrally molded, and said bottom membrane has a light-transparent property.

5. A culture device formed in a rectangular box shape from a deformable material, comprising a bottom membrane and side walls upstanding from the entire periphery of said bottom membrane, wherein a latching member which latches sample cells is formed on said bottom membrane.

6. A culture device according to claim 5, **characterized in that** said latching member is a protrusion.

7. A culture device according to claim 6, **characterized in that** said protrusion is porous.

8. A culture device comprising a deformable sample cell culture membrane, wherein a latching member which latches sample cells is formed said sample cell culture membrane.
